# EUROPEAN PATENT APPLICATION

(11) **EP 4 825 303 A1**
(43) Date of publication of application: **21.10.2026**
(21) Application number: 25171436.6
(22) Date of filing: 17.04.2025
(51) Int. Cl.: A61B 1/00, A61B 1/05, A61B 1/06, A61B 1/227, A61B 1/32

(54) **DEVICE FOR REMOTE OTORHINOLARYNGOLOGY (ORL) DIAGNOSIS**

(71) Applicant: ORLIASI SRL, 700309 Iasi (RO)
(72) Inventor: ILINCA, ANA MARIA, 700289 Iasi (RO); AVASILOAIEI, FLORIN ADRIAN, 700082 Iasi (RO)
(74) Representative: Petculescu, Ana-Maria

(57) **Abstract**

The invention relates to a device for remote otorhinolaryngology (ORL) diagnosis comprising a headset (2) intended to be placed on a patient's head, or to be manually hold by the patient, during use of the device. The device also comprises a telescopic speculum (4) releasably provided on the headset, intended to stretch a body cavity of the patient during use, and a camera (5), arranged in the telescopic speculum for filming within the body cavity wherein the telescopic speculum is inserted. A control unit (6) of the device controls the telescopic speculum and the camera and has communication means, for example a wireless communication interface, for communication with a remote otorhinolaryngologist, in particular with a mobile device of the specialist, in such a way that the specialist may remotely control the device, in particular the camera.

## Description

### Field of the Invention

The present invention relates to the field of medical diagnostic devices, and in particular relates to a device for performing otorhinolaryngology (ORL) diagnosis remotely, i.e. at distance from the patient, in real-time.

### Background

The expression otorhinolaryngology diagnosis, in short ORL diagnosis, refers to the diagnostic procedures and evaluations conducted by otorhinolaryngologists to identify and treat conditions affecting a patient's ear, nose, and throat. Otorhinolaryngologists are often referred to as ENT specialists, where ENT indicates, in fact, ear, nose and throat. ORL diagnoses can involve a variety of tests, including blood tests, X-rays, scans, but are mostly based on direct examinations of the vocal cords, the pharynx and the ears of the patient by the otorhinolaryngologist.

An otorhinolaryngologist can diagnose and treat conditions such as ear infections, hearing loss, tinnitus, vertigo, sinusitis, allergies, nasal obstruction, tonsillitis, and throat cancer. Otorhinolaryngologists also handle issues related to voice and swallowing disorders, and can perform both surgical and non-surgical treatments. Additionally, they manage head and neck cancers, facial trauma, and reconstructive surgery of the face, scalp, and neck.

It is not always possible to perform a traditional ORL diagnosis, with the otorhinolaryngologist directly examining the patient, in presence, as the patient may be located remote. This can be the case, for example, of patients living in remote areas far from hospitals or where an otorhinolaryngologist is not available, or research expedition members temporarily living in the wilderness, etc.

Solutions have been developed to perform remote ORL diagnoses.

US 11,471,035 discloses an ear ailment diagnostic device that comprises a pair of earpieces, which both further comprise a light source, a magnification lens, an air conduction channel and a miniature camera. Each earpiece is coupled to an air conduction tube, an insufflator and an electrical wiring/data tube which is coupled to a computer. The insufflator may be manually, electronically, or battery powered. The computer comprises a smart phone for data processing and wireless communication for remotely interpretation of the collected data by an otorhinolaryngologist.

US 2021/0358582 discloses multiple diagnostic devices for performing ORL diagnoses, collectively referred to as a telehealth system. Among the devices there is an over-ear device, that may have several configurations, intended to be placed by the patient over the ear for collecting data.

The Applicant has found that, albeit known devices do allow for examination of a patient's ear, nose, and throat, examination is generally cursory, as images that can be acquired may not detailed enough to simplify interpretation by the remote otorhinolaryngologist and maximize precision of the diagnoses. In particular, images mostly show mostly external areas of a patient's ears, nose and throat, not the most internal areas that are, in fact, those usually affected by a pathology.

Moreover, known devices are basically self-operated by the patient, meaning that it is the patient that manipulates the device to acquire images and, consequently, errors can be made by those who have no experience or, for example, cannot handle objects in their hands in a stable way.

### Summary of the Invention

The aim of the present invention is to provide a device for remote otorhinolaryngology (ORL) diagnosis that permits to remotely perform precise diagnoses, in real time, even when the patient handling the device has no experience.

The invention relates to a device for remote otorhinolaryngology (ORL) diagnosis comprising a headset intended to be placed on a patient's head, or to be manually hold by the patient, during use of the device. The device also comprises a telescopic speculum provided on the headset, intended to stretch a body cavity of the patient during use, and a camera, arranged in the telescopic speculum for filming within the body cavity wherein the telescopic speculum is inserted. A control unit of the device controls the telescopic speculum and the camera and has communication means, for example a wireless communication interface, for communication with a remote otorhinolaryngologist, in particular with a mobile device of the specialist, in such a way that the specialist may remotely control the device, as it will be disclosed in the following. As an optional feature at least one speaker can be arranged on the headset for transmitting to the patient the instructions of the otorhinolaryngologist.

### Definitions

The term *speculum* is used to indicate a component or module of the device that is intended to be inserted in a body cavity of the patient, such as the ear's canal, in order to reach a deep area of the cavity with the camera. Eventually, depending upon the dimensions of the body cavity, the speculum may stretch the cavity. For example, this might occur when, due to ear's infection, the ear canal is almost closed and any image taken from the outside of the canal is useless.

The term *telescopic,* referred to the speculum, indicates that the speculum can be extended from a configuration of minimum length to a configuration of maximum length. Extension can be selective, meaning that the speculum may be extended to a given length and that length is kept until the extension of the speculum is again changed, voluntarily. The expression *control unit* indicates any electronic circuit configured to exercise control over the components of the device, and in particular control by the remote otorhinolaryngologist. Control means, for example, switching on and off, extending or retracting the telescopic speculum, using the speaker, activating the communication means for remotely acquiring images from the camera, etc.

The proposed solution allows for performing effective examinations and, consequently, for providing precise ORL diagnoses, in real-time, in that the speculum brings the camera within the patient's body cavity to be examined, where images can be taken that are valuable source of information for the remote otorhinolaryngologist. Thanks to the fact that the telescopic speculum can be brought to the extension desired by the remote specialist, the body cavity of the patient can be inspected completely, for its entire length; for instance, the ear cavity can be completely inspected from the outside of the ear to the area of the eardrum, as well as the throat can be examined more in depth.

Not only the telescopic speculum brings the camera deep in the body cavity, but also helps stretching the cavity when insertion of the camera would be otherwise impossible or difficult. By inserting the telescopic speculum in a body cavity at its minimum length, and by extending the telescopic speculum, images can be taken of the body cavity along its extension covered by the camera lodged in the speculum. This allows the specialist to examine the entire cavity and not only the areas that are accessible from the outside of the cavity. The specialist can, therefore, evaluate the condition of the entire body cavity.

The device of the present invention is portable and handheld, meaning that is suitable for carrying on the patient's body and for handling with the hands.

The headset is to be considered a base or console onto which several accessories can be mounted in a modular way, as specified in the following.

The telescopic speculum can be motorized or manual.

In an embodiment, the telescopic speculum is motorized and is selectively extendable between a first configuration of minimum length to a second configuration of maximum length. The extension of the telescopic speculum is feedback controlled by the remote otorhinolaryngologist through the control unit, meaning that the extension can be adjusted to the desired length and maintained to such length for the desired amount of time. In this embodiment, for instance, the patient wears the headset on the head, with the telescopic speculum initially retracted (minimum length) and partially inserted in the ear to be examined. Upon establishing a connection with a remote otorhinolaryngologist, this specialist may take over control of the telescopic speculum and adjust its extension to insert the camera in the ear canal and acquire images or a live feed. The camera can be brought forward and backward by the otorhinolaryngologist. At the same time, the otorhinolaryngologist may talk to the patient, to impart instructions or simply to provide explanation to make the patient comfortable. The voice of the otorhinolaryngologist is heard by the patient through the speaker of the headset.

In another embodiment, the telescopic speculum is configured for manual operation and is selectively extendable by an user, such as the patient, a healthcare professional or another individual assisting the patient, between a first configuration of minimum length to a second configuration of maximum length. In this embodiment, the length of the telescopic speculum can be adjusted by the patient according to the instructions received from the otorhinolaryngologist over the speaker of the device. The examination of the ear canal, for instance, may imply more than one insertion of the telescopic speculum, in order to acquire images at different depths in the ear canal.

Preferably the telescopic speculum is interchangeable on the headset; in this respect, the telescopic speculum can be considered a module that can be attached to the headset and detached from the headset, according to the need. For example, the device can be configured with the telescopic speculum attached to the ear region of the headset in order to perform a patient's ear inspection but, if needed, the telescopic speculum can be separated from the headset and reattached to a different portion of the headset for performing examination of the oral cavity. Interchangeability brings the possibility to provide several telescopic speculums, having different dimensions, for coupling with the headset, and easiness of replacement.

In the preferred embodiment the telescopic speculum can be removably attached to the headset by means of a magnetic coupling; alternatively, a mechanical coupling can be set in place, for example a screw type coupling.

For supplying power to the speaker, the control unit and any module associated to the headset, the device can be powered by an external power source or may comprise at least one battery or rechargeable battery (accumulator), preferably arranged within the headset. When one of the modules is the motorized telescopic speculum, for instance, the telescopic speculum is powered by the battery as soon as a connection is established with the headset, i.e. as soon as the telescopic speculum is attached to the headset. The battery may be removable or replaceable. In a most preferred embodiment, the battery is built in the headband of the headset, requiring a custom shape to ensure a reasonable capacity within the available space. A removable battery would occupy additional volume and further limit the achievable capacity, making it less suitable for compact integration.

Preferably the telescopic speculum has a first end portion configured for connecting to the headset, for example magnetically or mechanically as anticipated above, and a second end portion, opposed to the first end portion. The camera is arranged in the second end portion, so that it can be displaced as the telescopic speculum is extended.

In the preferred embodiment In the preferred embodiment, the second end portion of the telescopic speculum is further configured to be tiltable with respect to the extension axis of the speculum, the tilting being actuated by an actuator. This arrangement enables angular adjustment relative to the extension/longitudinal axis of the speculum, facilitating improved access to the ear canal and providing an enhanced viewing angle. Preferably, the tiltable second end portion has a length of 1 cm or less, and the tilting angle is approximately ±3° relative to the extension axis. This configuration ensures safe operation of the device, minimizing the risk of injury to the ear canal, even when used by an inexperienced user, such as in self-assessment scenarios. Alternatively or further to the above the camera is orientable relatively to the telescopic speculum to permit examination of a body cavity around the extension axis of the telescopic speculum. The device comprises an actuator of the camera, preferably arranged within the telescopic speculum; the camera is tiltable by the actuator by an angle relatively to the extension axis of the telescopic speculum. The actuator can be a small electric motor.

The camera is remotely controlled by the otorhinolaryngologist through the control unit of the device, using a communication interface. In this way, the otorhinolaryngologist may adjust the orientation of the camera to focus on those areas of the patient's body cavity that require attention. In the embodiment wherein the telescopic speculum is motorized, the otorhinolaryngologist remotely controls both the extension of the telescopic speculum and the orientation of the camera (or its lens).

Preferably the device comprises a light source arranged on the second end portion of the telescopic speculum for illuminating a body cavity in front of the camera, during use of the device. For example, LED light sources can be used, emitting cold light.

In the preferred embodiment the device comprises a protective sleeve of the telescopic speculum, interchangeably fitted on the telescopic speculum. In order to accommodate the change of length if the telescopic speculum, also the sleeve is telescopic, or extensible. The protective sleeve isolates the telescopic speculum from the body of the patient, in particular from fluids, saliva, earwax, etc., and at the same time guarantees hygiene for the patient, since the telescopic speculum can be used for examination of ears, nose and throat one after the other.

For instance the protective sleeve is telescopic and has a first end portion configured for attachment to the headset, around the first end portion of the telescopic speculum, and a second end portion configured for attachment to the telescopic speculum, preferably at its second end portion, around the light sources. This magnetic coupling provides for easy interchangeability after any insertion in a body cavity or after contact with a body fluid.

Optionally the protective sleeve also protects the at least one speaker of the headset, meaning that the first end portion of the protection sleeve encompasses also the speaker when the protective sleeve is coupled to the headset.

Optionally, the device also comprises another module, an orientable or flexible arm, extending from the headset, for performing visual examination of the oral cavity or the nose. Although the arm can be used to support a microphone, in a conventional way for a headset, the same arm may be provided with an arm camera or with a distal support for attachment with the telescopic speculum. The arm camera, also remotely controlled by the otorhinolaryngologist, can be brought by the patient in front of the nostrils or partially inside the mouth, to take images of the nostrils or the throat. The otorhinolaryngologist imparts instructions to the patient over the speaker, and the patient follows the instructions to bring and orientate the camera as requested. The same achievement can be obtained by mounting the telescopic speculum on the orientable or flexible arm, in a similar way as it can be attached to, and detached from, the headset. Also, the orientable or flexible arm can be attached to, and detached from, the headset.

In an embodiment the device comprises another module: a dispensing unit, removably attachable to the headset and remotely controlled, for example by means of a magnetic or mechanical coupling, configured for dispensing a medical fluid. The dispensing unit is fillable with a medicament that can be released in the patient's body cavity upon command imparted remotely by the otorhinolaryngologist.

The device can be defined as modular device, in that the headset can assume different configurations depending upon the specific combination of modules, or accessories, installed on it. It serves the purpose of allowing remote ORL diagnoses even when the patient has no medical background or experience, as the remote specialist can talk to the patient and guide him/her through the entire examination procedure.

### Brief description of the drawings

Further characteristics and advantages of the invention will be more evident by the review of the following specification of a preferred, but not exclusive, embodiment, which is depicted for illustration purposes only and without limitation, with the aid of the attached drawings, wherein:
- Figure 1 is a perspective view of a device for remote ORL diagnosis according to the present invention;
- Figure 2 is perspective exploded view of a detail of the device shown in figure 1;
- Figure 3 is an exploded view of a component of the device shown in figure 1;
- Figure 4 is a schematic view of a detail of the device of the present invention;
- Figure 5 is a perspective view of another embodiment of the device for remote ORL diagnosis according to the present invention.

### Detailed description of the invention

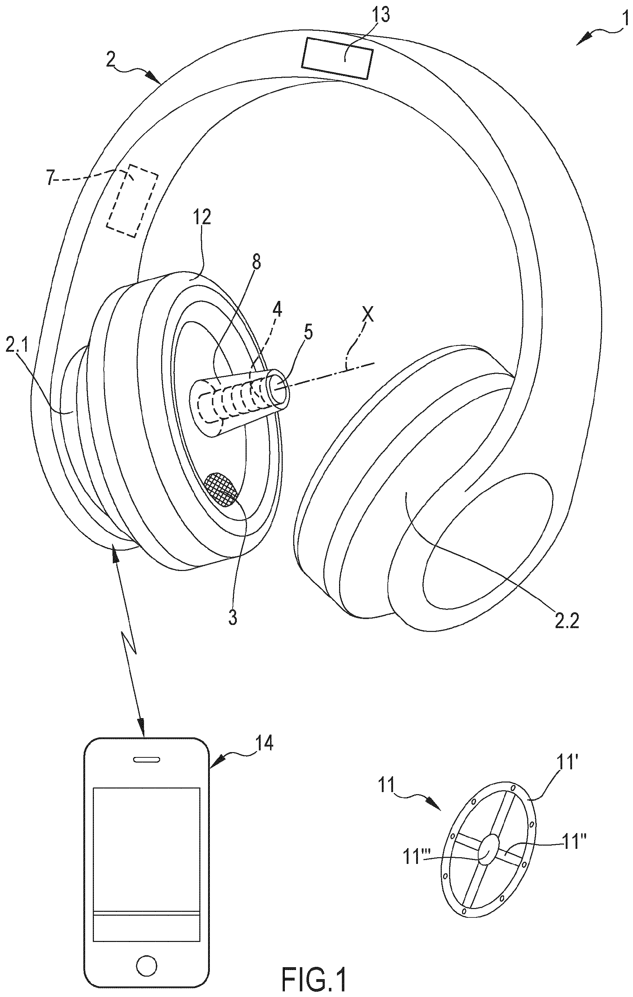

Figures 1 to 3 show the preferred embodiment of the device 1 according to the present invention, which is a portable medical device 1 that can also be handheld by a patient, that serves the purpose of allowing a remote otorhinolaryngologist to perform a ORL diagnosis of a condition of the patient's ears, nose or throat, in real-time, i.e. while the patient is handling or manipulating the device 1.

The device 1 comprises a headset 2 configured for wearing on the patient's head, wherein the head set comprises at least one ear portion 2.1, or two ear portions 2.1, 2.2 as shown in figure 1. In the at least one ear portion 2.1 a speaker 3 is arranged for listening to the otorhinolaryngologist or listening to a podcast or music (for relaxing during examination). A suitable speaker is, for instance, a 30 mm diameter audio speaker. A cushioned pad 12 surrounds the speaker 3, providing comfort and correct positioning of the ear portion. As it can be seen, the speaker 3 is not centered within the ear portion 2.1, but is instead shifted relatively to the center of the ear portion 2.1.

A first module 4, consisting of a telescopic speculum, can be releasably attached to the ear portion 2.1 of the headset 2, in such a way that the telescopic speculum 4 is coaxial with an axis X oriented perpendicularly to the ear portion 2.1 of the headset. The module 4 is located in the lower section of the ear portion 2.1, ensuring that, when the patient correctly wears the headset 2, the telescopic speculum is aligned with the patient's ear cavity.

A lens camera 5 is arranged within the telescopic speculum for taking images, videos, video feeds, once the telescopic speculum is placed in a body cavity of the patient, like a ear canal, or in front of a body cavity like the mouth or the nose nostrils.

A second module 9, which is optional, is a flexible or extendable arm 9 releasably attached to the ear portion 2.2 of the headset 2. The arm 9 has a distal portion 10 onto which a microphone is installed and/or another attachment can be established with the telescopic speculum 4. As alternative to the possibility of connecting the telescopic speculum 4, on the distal portion 10 another camera is arranged, named arm camera. The arm camera may be used for taking images of the patient's oral cavity while the headset 2 is correctly kept on the head.

A control unit 13 is arranged in the headset 2. The control unit 13, for instance a printed circuit board PCB, controls any module connected to the headset 2 and also manages the connection with a remote specialist through a built-in communication interface. Preferably the communication interface is wireless, as schematically shown in figure 1, and connects to a mobile device 14 of the patient for starting a phone or video call with the otorhinolaryngologist. A suitable control unit 13 is a Raspberry Pi^{™} Zero 2W. Any known communication protocol or interface may be used, like direct Wi-Fi, Bluetooth, etc. The same mobile device 14 is used to transmit videos and images taken by the camera 5 lodged in the telescopic speculum 4. If needed, a software can be downloaded and installed in the mobile device 14 to elaborate or pre-elaborate the videos or the images, for instance by orienting the images, adjusting colours and saturation, superimposing grids for tracing dimensions, etc.. Connection with the remote specialist is preferably in real-time, meaning that the specialist controls the device during the examination of the patient wearing the headset 2, so that the specialist can immediately diagnose ORL pathologies and assess the general condition of the ear, buccal or nasal cavity. If the control unit 13 is a Raspberry Pi^{™} remote transmission can be achieved using a streaming server, for example mjpg-streamer, motion, ffmpeg, accessible via a mobile device like phone/laptop with a browser: http://pi-IP:port. Optionally, a camera switching button can be added in the web interface.

The lens camera 5 is remotely controlled by the otorhinolaryngologist, through the control unit 13, once the remote connection is established. Controlling the camera 5 means switching on and off, adjusting the focus, zooming in and out, and preferably also means adjusting the orientation of the lens of the camera 5 relatively to the extension axis X of the telescopic speculum 4. In this embodiment, in fact, the lens of the camera 5 can tilt upon command received from the control unit 13, preferably by an angle of ± 3°. This feature allows the remote otorhinolaryngologist to examine the patient's body cavity around the axis X, at an angle, thereby widening the area covered by the camera 5. Tilting lens permit, in fact, to better examine the ear canal, that may differ in shape from one patient to another.

The camera 5 may be, for example, a camera suitable for surgical operation. Alternatively, in those embodiments where the telescopic speculum 4 must be particularly thin and cannot accommodate a lens camera, an optical fibre is used instead, with the optical fibre arranged in the headset 2, in a retractable way, and extending through the telescopic speculum 4.

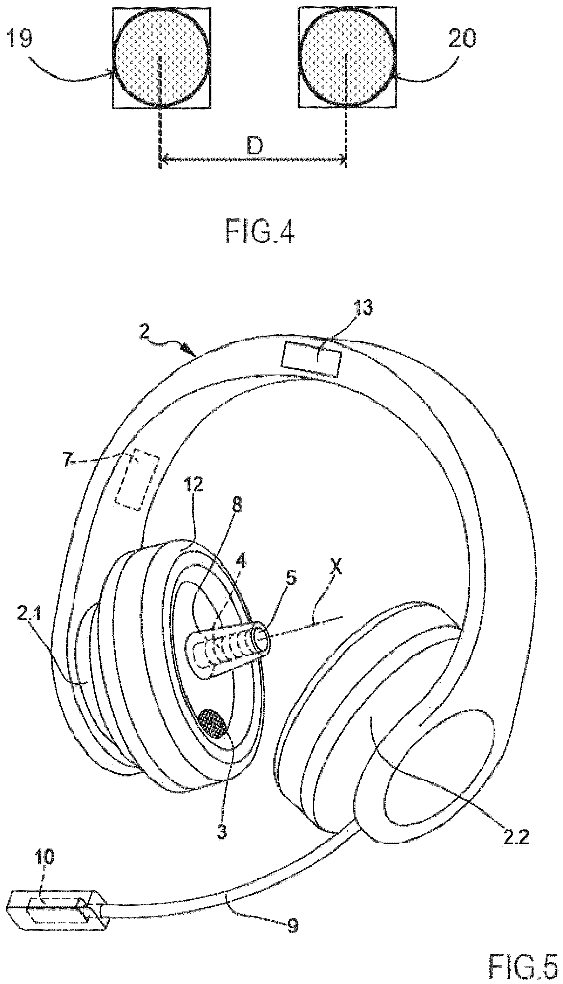

As shown in figure 4, preferably the camera 5 comprises two lenses 19, 20 arranged side by side, vertically or horizontally, within the telescopic speculum 4. A first lens 19 has fixed focus at about 1mm distance for providing close-up field of view; a second lens 20 has fixed focus at about 5 cm distance for providing mid-distance field of view. The two lenses 19, 20 may be arranged parallel, i.e. with focus on parallel lines of view, or slightly convergent by an angle of 5-10° towards a common point on the insertion axis X, where the remote specialist will focus examination. Preferably the two lenses 19, 20 are arranged at a distance D included in the range 1 - 1.5 mm, considering the distance between the optical centres of the lenses 19, 20. Suitable lenses 19, 20 preferably have dimensions of maximum 0.575 mm x 0.575 mm. Suitable lenses 19, 20 can be lenses developed by the company OMNIVISION Technologies with commercial name OV6948. Such lenses have an optical format of 1/36 inch and can capture 200 x 200 resolution video at up to 30 frames per second, and offer low-light sensitivity of 1000 mV/lux-sec.

Preferably camera lenses 19, 20 are mounted on a rigid board or in a 3D printed holder. Between or around lenses 19, 20 there can be left enough space to lodge LEDs, if necessary for providing sufficient illumination. Preferably one or two cool white LEDs are arranged, each connected with a resistor, also controlled by the control unit 13, for example a Raspberry Pi^{™}.

Switching between the feeds provided by the two lenses 19, 20 can be achieved using analog video switch (for example TI TS5A23157) controlled via the control unit 13 or with a physical/manual switch provided on the headset.

The camera 5 provides real-time video transmission (live stream), for example with analog video output, via Wi-Fi, accessible from browser/app of the mobile device 14.

Preferably the diameter of the camera 5 at the second end 4.2 of the telescopic speculum 4 is sufficiently small to allow to have 2 mm or less diameter of the second end 4.2 of the telescopic speculum 4. For example, the camera may have a diameter of less than 1.5 mm, and more preferably a diameter of approximately 0.6 mm. Preferably the telescopic speculum 4 has minimum length of about 1 cm, when completely retracted, and a maximum length of about 8 cm, when completely extended. In other embodiments, the minimum length may range between 0.5 cm and 2 cm, while the maximum extended length may range between 6 cm and 10 cm, depending on the specific application or anatomical considerations. These dimensions are merely illustrative and do not limit the scope of the invention.

Preferably an analog/digital converter is used to convert the analog feed provided by the camera 5. The converter is preferably a software installed on the mobile device 14 or on the control unit 13. For example, if a Raspberry Pi^{™} is used as control unit 13, a USB video grabber like EasyCAP^{™} is suitable.

Preferably, around the telescopic speculum 4 a protective sleeve 8 can be arranged, being interchangeably fixable onto the ear portion 2.1 of the headset 2. The protective sleeve 8 serves the purpose of protecting the telescopic speculum 4 and the camera 5 (or the optical fibre) from dirt, body fluid, ear wax, etc. It also serves hygienic purposes, as the telescopic speculum 4 can be used in the same examination procedure to investigate the ear, the nose and the throat. Due to the fact that the extension of the telescopic speculum 4 is variable, preferably in the range 2-8 cm, the protective sleeve 8 is configured to accommodate any extension of the telescopic speculum 4, for example by being itself telescopic, or by being extensible.

Reference number 11 indicates another module of the device 1 which is a dispenser of a medicament. The dispenser 11 is removably attachable to the headset 2 onto the ear portion 2.1 or, when such optional accessory is provided, onto the distal portion 10 of the arm 9, and is remotely controlled by the otorhinolaryngologist to spray or release the medicament upon command. Clearly, the device 1 can be sold in a kit with more than one dispenser 11, for example each for a different medicament.

The dispenser 11 is magnetically or mechanically (e.g. by screwing) attachable onto the ear portion 2.1 of the headset 2, for instance onto the magnetic ring 16 described below, just like other modules of the invention, or onto the second end 4.2 of the telescopic speculum 4.

Preferably, the dispenser 11 provides support for a pulverization attachment 11' made of a multilayer bag, laminated, which has two compartments: an active chamber that contains the medicine dose, preferably between 0.3 and 0.7 ml, and the pressure chamber that contains compressed air or gas, for instance nitrogen, that provides propulsion fluid for spraying the medicine. The multilayer bag is made of a flexible but strong material, composed of multiple layers of plastic, aluminium foil and/or extra barrier materials which will prevent oxidation or evaporation of the medicine.

By pressing the button 11‴, that preferably is located onto the ear portion 2.1 of the headset, the compressed air/gas reaches the pulverization attachment 11' through ducts 11" and causes the multilayer bag to rupture and release the medicine as a spray through a number of tiny holes preset in the bag. The button 11‴ can be pressed by the user or, alternatively, an electronic activation system is set in place for remote activation by the specialist. Medicine is sprayed into the body's cavity, exactly where it is needed, and with the correct dosing, under the supervision of the remote specialist.

When the device is provided with the optional arm 9, the dispenser 11 can also be removably attachable to the distal portion 10 of the arm 9, and is remotely controlled by the otorhinolaryngologist to spray or release the medicament into the oral cavity upon command.

The headset 2 has a seat for a battery 7 that is thereby inserted for powering any electrically powered modulus of the device 1, including the telescopic speculum 4, the speaker 3, any microphone on the arm 9 (if provided) and any dispenser 11. Preferably the battery 7 is rechargeable in its seat and also outside the headset 2, upon extraction from the seat. Preferably the battery 7 is a Li-Ion battery pack with capacity of about 3000 mAh.

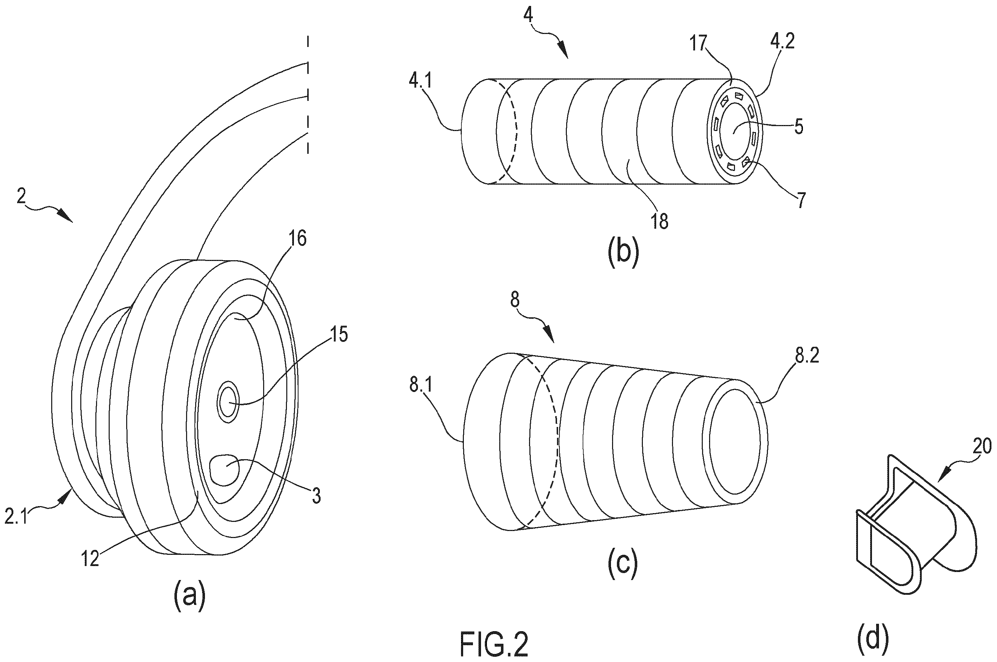

Figure 2 shows one ear portion 2.1 of the headset 2 and some of the modules that can be releasably attached to the headset 2.

In particular, in figure 2(a) reference number 15 indicates a contact electric plug that establishes an electric contact with the telescopic speculum 4 when the telescopic speculum 4 is brought into contact with the electric plug 15. The electric plug 15 also provides for a magnetic coupling with the first end portion 4.1 of the telescopic speculum 4 in such a way that coupling of the telescopic speculum 4 to the ear portion 2.1 of the headset 2 can be accomplished simply by bringing the first end portion 4.1 of the telescopic speculum 4 in contact with the electric plug 15, and separation can be achieved simply by pulling the telescopic speculum 4.

As shown in figure 2(b), the telescopic speculum 4 has a second end portion 4.2, opposite to the first end portion relatively to the extension axis X, onto which a source of light is provided. In the embodiment shown in the figures, the source of light counts several LEDs 7, preferably emitting cold light, for instance at 6000 Kelvin. LEDs are powered by the headset 2 through the electric plug 15. The camera 5 is arranged at the second end portion 4.2 of the telescopic speculum 4, with the consequence that the axial position of the camera 5 can be adjusted by remotely adjusting the extension of the telescopic speculum 4. The camera 5 is preferably a lens camera that is mounted onboard the telescopic speculum 4. The second end portion of the speculum 4.2 can be tilted, preferably by an angle of ±3° relatively to the extension axis X. Also the camera 5 is powered by the headset 2 through the electric plug 15 once the telescopic speculum 4 is magnetically coupled to the ear portion 2.1. The remote specialist uses this connection to remote controlling both the telescopic speculum 4 and the camera 5. Also, at the second end portion 4.2, around the LEDs 7, another magnetic ring 17 is arranged that provides for magnetic coupling of the second end portion 8.2 of the protective sleeve 8 to the second end portion 4.2 of the telescopic speculum 4. When the remote specialist switches on the camera 5, he/she may adjust axial displacement of the camera 5, as explained above, and can also turning on and off the LEDs 7 and tilt the camera 5 to bring it to the optimal orientation relatively to the body cavity to be examined.

The telescopic feature of the telescopic speculum 4 is preferably achieved using an electric motor (not shown) installed onboard of the telescopic speculum 4, that imparts rotations to the second end portion 4.2 around the axis X, while the first end portion 4.1 is kept stationary relatively to the same axis. By providing threads onto intermediate components 18 of the telescopic ring, rotation of the second end portion 4.2 can be set to cause axial movement of the second end portion 4.2, according to a known mechanical configuration. Alternatively, the telescopic speculum is manufactured like a conventional photo camera zoom lenses, motorized or manual.

Around the electric plug 15 a magnetic ring 16 is provided, that may encompass the speaker 3 as shown in figures 2(a) and 2(c), that provides a magnetic attachment to the protective sleeve 8, in such a way that coupling of the protective sleeve 8 to the ear portion 2.1 of the headset 2 can be accomplished simply by bringing the first end portion 8.1 of the protective sleeve 8 in contact with the magnetic ring 16, and separation can be achieved simply by pulling the protective sleeve 8. Therefore, the protective sleeve 8, when correctly installed, extends from the entire length of the telescopic speculum 4 and configure a sealed coupling at end portions 4.1 and 4.2 that prevents body fluids, dirt, ear wax and the like from reaching the side surface of the telescopic speculum 4. The protective sleeve 8 can be made of an elastically extensible film or of a folded film that can be unfolded, so as to accommodate for any extension imparted to the telescopic speculum 4; preferably the film is transparent and has a medical grade. Alternatively, also the protective sleeve 8 can be made telescopic, with intercoupling intermediate sections, that are extended when pulled by the second end portion 4.2 of the telescopic speculum 4.

In figure 2(d) another module is shown, a clip-on attachment 20 for mouth use. The clip-on attachment 20 is intended to be attached in a removable way onto the telescopic speculum 4, in such a way to remain ahead of the camera 4. Preferably, the clip-on attachment 20 is magnetically attachable onto the second end portion 4.2 of the telescopic speculum 4 or it can be locked onto the second end portion 4.2 of the telescopic speculum 4 by means of a mechanical coupling, for instance a screw type coupling. Once coupled to second end portion 4.2 of the telescopic speculum 4, the clip-on attachment 20 cantileverly extends in front of the camera 5, as a prolongation of the speculum 4, and provides a support for the patient teeth and lips, meaning that the patient may bite the clip-on attachment 20 to allow for examination of the oral cavity by means of the camera 5. Basically, the clip-on attachment 20 permits to keep the camera 5 stable, because the first end portion 4.1 of the telescopic speculum 4 is kept stable by the arm 9 or by the patient steadily handling the headset 2, and the second end portion 4.2 of the telescopic speculum 4 is kept stable by the patient biting the clip-on attachment 20.

As shown in figure 2(a), preferably the headset 2 can be divided in two halves, so that the patient can keep on half on the head and handle the other half to bring the camera 5 in front of the mouth or the nose, for examination of the oral cavity and nasal cavities. In an alternative configuration, the headset 2 may be additionally equipped with a fixing head piece (not illustrated in the figures), which can be secured on the user's head and rotatably coupled to the headset 2. This arrangement allows the headset to rotate relative to the fixed head piece, enabling the camera 5 to be positioned in front of the user's mouth or nose, while maintaining the overall stability and correct positioning of the device during use.

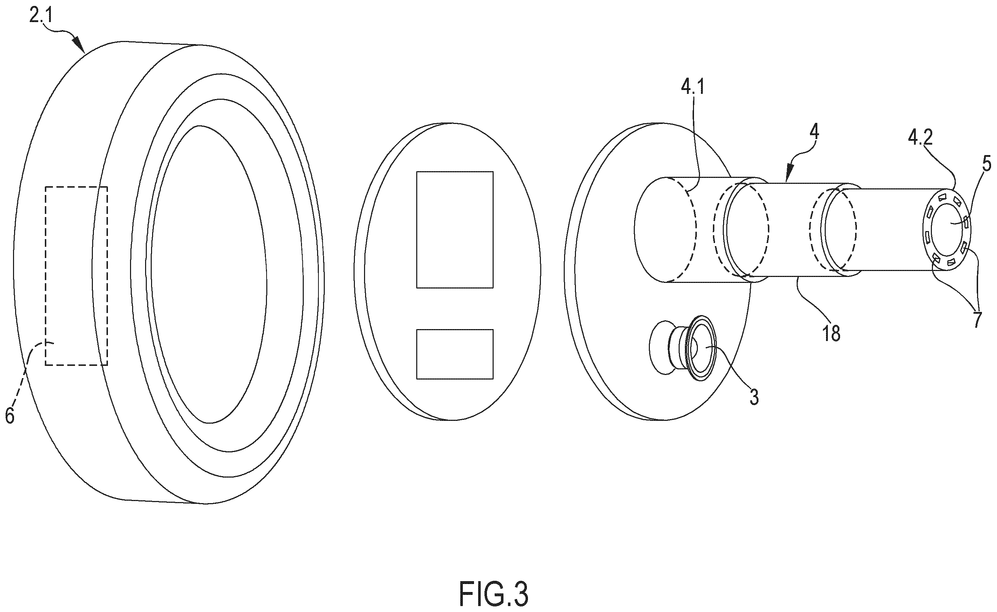

Figure 3 shows an exploded view of the ear portion 2.1 of the headset 2. Reference number 6 schematically indicates the control unit 6, that allows the remote wireless connection with the otorhinolaryngologist, and controls the camera 5, the speaker 3, the telescopic speculum 4 and the dispenser 11. As anticipated above, the control unit 6 is preferably a printed circuit board PCB with a wireless communication interface and a programmable unit programmed to impart commands to the controlled modules, depending upon the orders remotely imparted by the otorhinolaryngologist.

Functioning of the modular device 1 of the present invention will be now described with reference to various examinations.

### Oral Cavity Evaluation Module

For examining the oral cavity, the device 1 can be used in two ways. In a first configuration the headset 2 can be held with one hand in front of the patient's mouth, with the patient biting the clip-on attachment 20 and the camera 5 oriented towards the patient's throat above the tongue. As described above, the remote specialist remotely controls the camera 5 to perform examination of the oral cavity. Eventually, in this first configuration, the headset 2 is divided in two halves and one half is kept on the head, while the other half with ear portion 2.1 is positioned as described, with the camera 5 in the patient's mouth. In a second configuration, corresponding to the embodiment with the optional arm 9, the headset 2 is kept on the patient's head and the arm 9 is brought by the patient in front of the mouth; examination is carried out by attaching the telescopic speculum to the distal portion 10 of the arm 9, or using the arm camera.

### Nasal Cavity Evaluation Module

For examining the nasal cavity, the device 1 can be used in two ways, similarly to the evaluation of the oral cavity. In a first configuration the headset 2 can be held with one hand in front of the patient's nose, in order to have the camera 5 framing the nostrils. If the telescopic speculum 4 is sufficiently thin, the camera 5 may be brought at least partially inside the nostrils. In a second optional way, the arm camera is used by bringing the distal portion 10 just in front of the nostrils.

### Localized Treatment Application Module

The device 1 may also be used to release a medicament. The device 1 can be configured with the dispenser attached to the ear portion 2.1 of the headset 2 or to the distal portion 10 of the arm 9. Once the connection with the remote specialist has been established, the specialist may command the dispenser 1 to release the medicament in the ear canal, or in the nose nostrils or in the throat. Releasing the medicament may include, for example, spraying the medicament, or releasing drops of the medicament.

## Claims

1. A device (1) for remote otorhinolaryngology (ORL) diagnosis comprising:
- a headset (2) for wearing on a patient's head, or to be manually hold by the patient, during use, wherein the headset (2) comprises at least one speaker (3);
- a telescopic speculum (4) provided on the headset (2);
- a camera (5), arranged in the telescopic speculum (4);
- a control unit (6) of the device (1), wherein the control unit (6) has communication means for communication with a remote otorhinolaryngologist.

2. Device (1) according to claim 1, wherein the device (1) is portable and handheld.

3. Device (1) according to claim 1 or claim 2, wherein the telescopic speculum (4) is motorized and is selectively extendable between a first configuration of minimum length to a second configuration of maximum length, and extension of the telescopic speculum (4) is feedback controlled by the remote otorhinolaryngologist through the control unit (6).

4. Device (1) according to claim 1 or claim 2, wherein the telescopic speculum (4) is manual and is selectively extendable by an user between a first configuration of minimum length to a second configuration of maximum length.

5. Device (1) according to any of the preceding claims, wherein the telescopic speculum (4) has a first end portion (4.1) configured for connecting to the headset (2) and a second end portion (4.2) opposed to the first end portion (4.1), wherein the camera (5) is arranged in the second end portion (4.2).

6. Device (1) according to claim 5, wherein the second end portion (4,2) is orientable relatively to the telescopic speculum (4) and/or wherein the camera (5) is orientable relatively to the telescopic speculum (4).

7. Device (1) according to claim 6, further comprising an actuator of the second end portion (4,2) and/or of the camera (5), and wherein second end portion (4,2) and/or of the camera (5) is tiltable by the actuator by an angle relatively to the extension axis (X) of the telescopic speculum (4).

8. Device (1) according to claim 7, wherein the actuator is remotely controlled.

9. Device (1) according to any of the preceding claims, wherein the telescopic speculum (4) is interchangeable on the headset (2).

10. Device (1) according to claim 9, wherein the telescopic speculum (4) is removably attached to the headset (2) by means of a magnetic coupling or a mechanical coupling.

11. Device (1) according to any of the preceding claims, wherein the camera (5) comprises two lenses (19, 20) arranged side-by-side and having different fixed focus one relatively to the other.

12. Device (1) according to any of the preceding claims 8-12, comprising a light source (7) arranged on the second end portion (4.2) of the telescopic speculum (4), wherein the light source (7) emits light in a body cavity in front of the camera (5) during use.

13. Device (1) according to any of the preceding claims, comprising an orientable or flexible arm (9) extending from the headset (2), wherein the arm (9) is provided with an arm camera (10) or with a distal support (10) for attachment with the telescopic speculum (4).

14. Device (1) according to any of the preceding claims, comprising a dispensing unit (11), removably attachable to the headset and remotely controlled, configured for dispensing a medical fluid.

15. Device (1) according to any of the preceding claims, wherein the headset (2) is a base or consol onto which the telescopic speculum (4) and other accessories are attachable in a modular way.
